# EUROPEAN PATENT APPLICATION

(11) **EP 2 053 061 A1**
(43) Date of publication of application: **29.04.2009**
(21) Application number: 09152902.4
(22) Date of filing: 05.12.2002
(51) Int. Cl.: C07K 14/535, C07K 1/22, A61K 38/19

(54) **PROCESS FOR THE PURIFICATION AND/OR ISOLATION OF BIOLOGICALLY ACTIVE GRANULOCYTE COLONY STIMULATING FACTOR**

(30) Priority: 19.12.2001 SI 200100322
(62) Divisional of application: 02804876.7
(71) Applicant: Lek Pharmaceuticals D.D., 1526 Ljubljana (SI)
(72) Inventor: Gaberc Porekar, Vladka, 1526, Ljubljana (SI); MENART, Viktor, deceased (SI)
(74) Representative: Roeser, Tobias Karsten

(57) **Abstract**

The invention relates to the process for the isolation of biologically active granulocyte colony stimulating factor (G-CSF), which enables the separation of correctly folded biologically active monomeric molecules of G-CSF from the incorrectly folded, biologically inactive monomeric, oligo- or polymeric and also from aggregated molecules of G-CSF by using immobilised metal affinity chromatography. The process of the invention, if desired the whole process, can be advantageously performed under native conditions. The biologically active G-CSF with a purity of greater than 95% is thus obtained. Only two additional chromatographic steps, cationic exchange chromatography and gel filtration, are then preferably applied to remove the traces of impurities. The entire process results in the production of higher yields of G-CSF with a purity of greater than 99%. The described process is particularly suitable for the industrial production of G-CSF.

## Description

### Field of the invention

The invention relates to a new process for the purification and/or isolation of biologically active granulocyte colony stimulating factor (G-CSF) by using the immobilised metal affinity chromatography (IMAC).

G-CSF belongs to the group of colony stimulating factors which regulate the differentiation and proliferation of hematopoietic precursor cells and activation of mature neutrophils. G-CSF is used in medicine in the field of hematology and oncology. Two types of G-CSF are clinically available: a glycosylated form (lenograstim) which is produced by using the expression in mammalian cells, and nonglycosylated form (filgrastim) which is produced by using the expression in bacteria *Escherichia coli (E. coli*).

### Background of the invention

The nonglycosylated form of G-CSF (filgrastim) and its production are described in EP 237545 whereas the glycosylated form of G-CSF (lenograstim) and its production are described in EP 169566.

The processes for purification and/or isolation of G-CSF, which are known from the patent and scientific literature comprise different combinations of ion chromatography, chromatofocusing, hydrophobic interaction chromatography, gel filtration and some other methods.

Generally, the first step of the process for purification and/or isolation of G-CSF depends on the host organism of the heterologous expression of G-CSF. In case of the expression in *E. coli,* G-CSF is found in the insoluble inclusion bodies. The conventional processes therefore comprise additional steps for the isolation of G-CSF from the inclusion bodies leading to correctly folded biologically active form. These steps usually comprise washing with detergents or chaotropic substances, solubilisation with strong denaturing agents (e.g. guanidine hydrochloride (GndHCl), urea) or with high concentrations of detergents (e.g. N-lauroyl-sarcosine (sarcosyl) or sodium dodecyl sulfate (SDS)), partial purification of solubilised denatured protein by using gel filtration, reverse phase HPLC (RP-HPLC) and renaturation by using dilution of the denaturating agent or by using dialysis. In case of the expression of G-CSF in yeast or mammalian cells the inclusion bodies or similar structures are found rarely and the process for the purification and/or isolation in these cases starts directly after the secretion of G-CSF. The processes for the purification and/or isolation of G-CSF are described in the following patent applications and patents: EP 169566, EP 237545, EP 215126, EP 243153, US 5055555 and WO 0104154. The processes for the purification and/or isolation of G-CSF are also described in the scientific literature: Lu, H.S. et al. in Protein Expr Purif 4, 465-472 (1993), Kang, S.H. et al. in Biotechnol. Lett. 17, 687-692 (1995), Wingfield, P et al in Biochem J 256, 213-218 (1988), Kang, S.H. et al. v Biotechnol. Lett. 17, 687-692 (1995), Yamasaki, M. et al in Biosci Biotechnol Biochem 62, 1528-1534 (1998), Wingfield, P. et al. in Biochem J 256, 213-218 (1988), Bae, C.S. et al. in Biotechnol. Bioeng. 57, 600-609 (1998).

In case of some other proteins IMAC has also been used for partial purification and renaturation. IMAC was firstly described in Porath et al. in Nature 258, 598-599 (1975) and is based on the binding of proteins to immobilised metal ions, which are chelated to various IMAC supports. The electron donor groups in the amino acid sequence of the protein are responsible for the co-ordinate binding to the support, especially the imidazole ring in the histidine residues. The isolation of recombinant proteins with engineered histidine affinity tags on either N- or C- termini of the protein by using the IMAC was described by Hochuli, E. et al. in Bio/Technology 1321-1325 (1988), Chaga, G. et al. in Biotechnol Appl Biochem 29 (part 1), 19-24 (1999) and Jeong, J.K. and Lee S.Y. Protein Expr. Purif, 23: 311-318 (2001). The use of IMAC for the investigation of small topographical differences among protein molecules was described by Sulkowski in Trends Biotechnol 3, 1-7 (1985) and by Hemdan et al. in Proc Natl Acad Sci U S A 86, 1811-1815 (1989).

The process for the purification by using IMAC of some other proteins which comprise histidine residues was described in US 5932102.

The process for the purification of proteins with surface amino acids capable of binding metal ions is described in WO 9012803. In this process IMAC is used as an additional step after partial protein purification by using several other chromatographic methods. Neither isolation nor separation of non-denaturated or biologically active molecules of G-CSF from the denaturated or biologically inactive molecules of G-CSF under native conditions by using IMAC are described.

Comparative studies of G-CSF, its Ser-17 and (His)₆-tagged forms interaction with metal ions by means of affinity partitioning to a specific dye-metal ion complex were described (Zaveckas, M. et al. in J Chromatogr A 904, 145-169 (2000). Based on the evaluation of the chromatographic behaviour of bromelain and pure G-CSF on metal-free and Hg(II) charged IDA (iminodiacetate) columns, Gelunaite, L. et al. in J Chromatogr A 904, 131-143 (2000) made attempts to evaluate the ability of Hg(Il)-charged IMAC (Sepharose IDA) to extract G-CSF under denaturating conditions from detergent-solubilized inclusion bodies.

IMAC with immobilised Zn (II) or Ni (II) ions was also used as a method for renaturation of GndHCl denaturated interleukin-3, G-CSF and granulocyte macrophage colony factor (GM-CSF) (Rozenaite, V. et al. in Poster abstract , P-104., Cordoba, Spain, 19-22. April (1998)). The denatured proteins were bound to the IMAC support under denaturating conditions.

### Summary of the invention

It is an object of the invention to improve the purification and/or isolation of G-CSF, and to provide biologically active G-CSF in highly purified and active form, as well as a pharmaceutical composition comprising the same.

The present invention provides a process for the purification and/or isolation of biologically active G-CSF according to claim 1. The present invention further provides a process for the purification and/or isolation of biologically active G-CSF according to claim 27, a biologically active G-CSF according to claim 31, a pharmaceutical composition according to claim 33 and the use of a biologically active G-CSF according to claim 35. Preferred embodiments are set forth in the dependent claims.

According to the present invention it was surprisingly found that correctly folded and biologically active molecules of G-CSF can be separated from incorrectly folded or biologically inactive molecules of G-CSF under native conditions by using IMAC. The process for purification and/or isolation of biologically active G-CSF of the present invention comprises the separation of correctly folded and biologically active molecules of G-CSF from incorrectly folded or biologically inactive molecules of G-CSF and also from the majority of host proteins by using IMAC under native conditions. By this process the correctly folded and biologically active molecules of G-CSF specifically bind to the IMAC support whereas the incorrectly folded, biologically inactive forms of G-CSF and the majority of the impurities remain in the eluate. The process for purification and/or isolation of the present invention also comprises the separation of the biologically active monomeric forms of G-CSF from oligomeric, polymeric and biologically inactive monomeric forms of G-CSF. The biologically active monomeric forms of G-CSF are specifically bound to the IMAC support whereas oligomeric, polymeric and biologically inactive monomeric forms of G-CSF essentially remain in the eluate.

The process of the present invention presents an effective step of purification and concentration of correctly folded biologically active monomeric forms or molecules of G-CSF and can be used as the key step in the entire purification and/or isolation process of G-CSF.

The process for purification and/or isolation of the present invention can be used in cases where G-CSF after the expression is secreted directly via the secretion pathway to the medium, or where G-CSF is formed in the form of inclusion bodies in the cytoplasm, periplasm or any other cell organelle. It can also be used for the purification and/or isolation of biologically active G-CSF directly from the solubilised inclusion bodies and in all cases where G-CSF had previously been denatured and then renatured. The process of the purification and/or isolation of biologically active G-CSF of the present invention can be maintained under native conditions all along the process.

The process for purification and/or isolation of biologically active G-CSF of the present invention results in the production of biologically active G-CSF with a purity of greater than 95%. Only two additional chromatographic steps, cationic exchange chromatography and gel filtration, which are applied in a preferred embodiment of the present invention, can further be used for the removal of traces of the remaining impurities (polishing).

The entire process of the present invention therefore leads to the production of biologically active G-CSF with a purity of greater than 99%. The process is suitable for the production of large quantities of biologically active G-CSF and is suitable for the industrial production of biologically active G-CSF.

A description on the separation of biologically active, monomeric, correctly folded molecules of G-CSF from oligomeric, polymeric or biologically inactive monomeric forms or incorrectly folded molecules of G-CSF by using IMAC under native conditions is not found either in the scientific or in the patent literature.

There are also no descriptions in the prior art of purification and/or isolation of biologically active G-CSF from a crude solution or mixture containing biologically active G-CSF molecules and impurities by binding of the correctly folded biologically active monomeric forms of G-CSF (i.e. those molecules which are already found in the solution or the mixture) to the IMAC support under native conditions. Additionally, the separation of molecules of G-CSF according to their conformational state by using IMAC has not been described.

All methods for purification and/or isolation of G-CSF found in the prior art comprise several steps. The use of only one chromatographic step as for separation of G-CSF according to its biological activity and correct folding and as well for the separation from impurities present in the solution or mixture and also as an effective method for concentration of G-CSF and production of biologically active G-CSF with a purity of greater than 95%, so that only additional polishing is necessary, has not been described in prior art.

### Detailed description of the invention and preferred embodiments thereof

The present invention relates to the use of IMAC as an effective chromatographic method in the process for purification and/or isolation of biologically active G-CSF. The correctly folded biologically active monomeric forms or molecules of G-CSF are selectively bound to an IMAC support under native conditions whereas the incorrectly folded or aggregated molecules of G-CSF and most of other impurities, particularly the solubilized proteins from inclusion bodies after expression from e.g. *E. coli* are essentially not bound to these supports and are eluted without being retained. This feature is supposed to occur due to specific distribution of natural histidine residues.

The term 'native conditions' used herein refers to the conditions by which the molecule (G-CSF protein) preserves the native conformation and the biological activity.

The term 'denaturing conditions' refers to the conditions by which the native conformation of G-CSF protein is not preserved, the biological activity is changed and is not preserved.

The term 'aggregated molecules' used herein refers to the molecules which form clusters of molecules held together by hydrophobic or also some other interactions (e.g. disulphide bonds). These molecules are not biologically active.

The term 'elution' used herein refers to washing or extraction of the adsorbed material from the chromatographic column.

The term 'eluate' used herein refers to the solution which is obtained by washing and extraction from the chromatographic column.

The term 'inclusion bodies' used herein refers to insoluble compact aggregates of incorrectly folded or partially correctly folded proteins.

The term 'inclusion bodies solution' used herein refers to the solution which comprises inclusion bodies.

The term 'biologically active G-CSF' used herein refers to G-CSF which is capable of promoting the differentiation and proliferation of hematopoietic precurser cells and the activation of mature cells of the hematopoietic system.

The term 'biologically active form (or molecule) of G-CSF' used herein refers to a form or molecule of G-CSF which is in a monomeric and non-denatured state and which is capable of providing the aforementioned biological activity.

The term 'impurity' used herein refers to a substance which differs from the biologically active molecule of G-CSF such that the biologically active molecule of G-CSF is not pure. The impurity may include at least one substance from the group consisting of biologically inactive monomeric forms and incorrectly folded molecules of G-CSF, oligomeric and polymeric forms of G-CSF, denaturated forms of G-CSF and host cell proteins. The impurity may also include further host cell substances such as DNAs, (lipo)polysaccharides etc., and additives which had been used in the preparation and processing of G-CSF.

The purity indicated herein refers to HPLC purity.

The process for purification and/or isolation of biologically active G-CSF of the present invention is particularly defined by comprising the following steps:
a) loading a solution or mixture, which comprises the biologically active form of G-CSF and an impurity, on the IMAC support;
b) selective binding of biologically active form of G-CSF to the IMAC support, optionally washing the IMAC column; and
c) eluting the biologically active form of G-CSF from the column
   The process of the present invention can be advantageously performed under native conditions.
   The process for purification and/or isolation of biologically active G-CSF of the present invention can additionally comprise further purification of biologically active G-CSF and preferably comprises the following steps which are performed after the purification and/or isolation of biologically active G-CSF by IMAC:
d) cationic exchange chromatography and/or
e) gel filtration.

The entire process of the present invention results in the production of biologically active G-CSF suitable for clinical use in medicine.

A biologically active G-CSF suitable for clinical use in medicine can already be obtained in an efficient and preferable manner by applying a purification and/or isolation process wherein an impure mixture containing G-CSF under native conditions is subjected to chromatographic step(s) which consist only of IMAC and optionally at least one of the purification methods selected from ion exchange and gel filtration techniques. The process of the present invention can also be used in case of purification and/or isolation of G-CSF derivatives such as methionyl G-CSF (Met-G-CSF), glycosylated, enzymatically or chemically modified (e. g. pegylated) G-CSF, G-CSF analogues and the fusion proteins which comprise G-CSF.

Significant advantages of the process for purification and/or isolation of the present invention include that:
1. the molecules of G-CSF are separated according to their conformational state and therefore according to their biological activity,
2. the process enables binding of biologically active molecules of G-CSF to the IMAC support under native conditions and consecutively the separation of biologically active molecules of G-CSF from biologically inactive molecules of G-CSF under native conditions,
3. the process enables binding of correctly folded molecules of G-CSF to the IMAC support under native conditions and consecutively the separation of correctly folded molecules of G-CSF from the incorrectly folded molecules of G-CSF under native conditions,
4. the process enables binding of biologically active monomeric forms of G-CSF to the IMAC support under native conditions and consecutively the separation of biologically active monomeric forms of G-CSF from the biologically inactive monomeric forms of G-CSF,
5. the process enables binding of monomeric forms of G-CSF to the IMAC support and consecutively the separation of monomeric forms of G-CSF from the oligo- and polymeric forms of G-CSF under native conditions,
6. the process enables the separation under native conditions of correctly folded monomeric biologically active molecules of G-CSF from other proteins and impurities which are present in the solution, mixture or medium,
7. with the process of the present invention, it is possible to significantly increase the specific activity of the purified G-CSF, for example to a range of specific activity of at least 1x10⁷ IU/mg, more preferably to a range of specific activity 7-8x10⁷ IU/mg, most preferably to a range of specific activity of about 1x10⁸ IU/mg, wherein the specific activity is measured by a method based on stimulation of cellular proliferation as described in example 5.
8. with the process of the present invention it is possible to produce G-CSF at high yield and with a purity of at least 95% or, when further comprising the purification with cationic exchange chromatography and gel filtration according to the preferred embodiment, even at least 99%, and therefore the process is suitable for the industrial production of biologically active G-CSF,
9. the entire process according to the preferred embodiment for purification and/or isolation of biologically active G-CSF, which comprises further purification by cationic exchange chromatography and gel filtration, does not require any additional steps of purification of G-CSF and can be advantageously maintained under native conditions all along.

The entire process for purification and/or isolation of biologically active G-CSF of the present invention is most preferably maintained under native conditions.

The process of the present invention is not a renaturation process of incorrectly folded molecules G-CSF, but is a process which comprises the specific binding to the IMAC support of the non-denatured or correctly folded biologically active monomeric molecules of G-CSF, which are already present in a solution, a mixture or a medium containing non-purified G-CSF.

The separation of monomeric from oligo- and polymeric forms of G-CSF occurs in such a way that the biologically active monomeric forms of G-CSF are bound to the IMAC support, whereas the oligo and polymeric forms, which can also occur in the aggregate form, essentially remain in the eluate.

Instead of IMAC, gel filtration could be used for the separation of monomeric form of G-CSF from oligo- and polymeric forms of G-CSF. The advantage of IMAC over the gel filtration is its concentration capability, higher binding capacity and the ability of separating the correctly folded monomeric forms of G-CSF from the incorrectly folded monomeric forms of G-CSF. Correctly folded monomeric forms of G-CSF cannot be separated from the incorrectly folded monomeric forms of G-CSF by gel filtration. Thus, by using IMAC better yields are obtained.

Another advantage of the process for purification and/or isolation of biologically active G-CSF of the present invention over other procedures known from the prior art is that (under the native conditions maintained all along the process) the isolation of correctly folded molecules of G-CSF from the mixture of different proteins and also from the mixture of molecules G-CSF occurring in different conformational states is enabled. Therefore a direct isolation of G-CSF from a (preferably diluted) culture medium or, particularly, from the inclusion bodies under native conditions is possible.

Additional advantages of the process of the present invention over conventional processes are also: the possibility to reduce or omit the use of detergents, the possibility to work in the absence of denaturating agents, which are either toxic or environmentally unfavourable (e.g. GndHCl or urea), and the possibility to reduce or omit the use of buffers and other solutions.

The advantage of the process for purification and/or isolation of biologically active G-CSF of the present invention over the methods in which strong denaturing agents are used is that there is no need of use of active reducing agents like dithiothreitol or beta-mercaptoethanol.

In case of secretion of G-CSF directly into the medium, the process for purification and/or isolation of biologically active G-CSF of the present invention enables effective and direct concentration of biologically active G-CSF from the diluted media. In the eluate from the IMAC column biologically active G-CSF of high purity is obtained.

Since the effective separation of the biologically active G-CSF molecule from biologically inactive G-CSF molecules and other impurities can be obtained either by the isolation of the biologically active G-CSF molecule from the inclusion bodies and/or by the isolation of the biologically active G-CSF molecule directly from the solution or the mixture containing it, the economy of the purification and/or isolation of G-CSF is much improved when compared with other methods.

G-CSF purity of more than 95% can thus be obtained by using only one purification step.

The following chromatographic step(s) is (are) particularly suitable for the final purification (polishing) of biologically active G-CSF after eluting from IMAC.
- Cationic exchange chromatography and/or
- Gel filtration

Cationic exchange chromatography is especially effective for removal of traces of nucleic acids, lipopolysaccharides and proteins derived from host cells, and for removal of ionic isomers of G-CSF and changed (damaged) forms of G-CSF with altered pl values. Gel filtration chromatography is especially effective for removal of traces of dimers and higher aggregated forms of G-CSF.

Using only the additional two final purification steps results in a purity of biologically active G-CSF greater than 99%.

The advantages of the entire process for purification and/or isolation of biologically active G-CSF according to a preferred embodiment of the invention, which additionally comprises the cationic exchange chromatography and gel filtration are: besides appropriate pre-processing steps, such as a solubilisation and an optional subsequent solubiliser removal by dialysis, ion exchange, ultrafiltration, diafiltration or dilution or the like, the process may efficiently comprise only the above specified three chromatographic steps; between the three chromatographic steps there are preferably no intermediary steps (like concentration, dialysis, precipitation, etc.), and the native conditions are preferably maintained all along the purification and/or isolation process.

The intermediary concentration steps would disadvantageously cause the formation of dimers and other forms of aggregates, leading to reduced yields. The entire process for purification and/or isolation can be transferred to industrial scale and to the production of biologically active G-CSF with a purity of at least 99%.

The biologically active G-CSF obtained by the entire process for the purification and/or isolation of the present invention is suitable for the preparation of pharmaceutical composition, which comprises the therapeutically effective amount of biologically active G-CSF and is suitable for clinical use.

The possibility of maintaining the active form of G-CSF in a short purification and isolation process contributes not only to an improved yield, but also to an improved purity and effectiveness of the biologically active G-CSF and the pharmaceutical composition containing it.

The term 'therapeutically effective amount' used herein refers to the amount of biologically active G-CSF which has the therapeutic effect of biologically active G-CSF.

Suitable pharmaceutically acceptable auxiliary substances include suitable diluents, adjuvants and/or carriers useful in G-CSF therapy.

Biologically active G-CSF which was obtained by using the process of the present invention, particularly when performing the additional steps of cationic exchange chromatography and gel filtration, can be used for preparation of medicaments, which are indicated for the indications selected from the group, which comprises: neutropenia and neutropenia-related clinical sequelae, reduction of hospitalisation for febrile neutropenia after chemotherapy, mobilisation of hematopoietic progenitor cells, as alternative to donor leukocyte infusion, chronic neutropenia, neutropenic and non-neutropenic infections, transplant recipients, chronic inflammatory conditions, sepsis and septic shock, reduction of rist, morbidity, mortality, number of days of hospitalisation in neutropenic and non-neutropenic infections, prevention of infection and infection-related complications in neutropenic and non-neutropenic patients, prevention of nosocomial infection and to reduce the mortality rate and the frequency rate of nosocomial infections, enteral administration in neonates, enhancing the immune system in neonates, improving the clinical outcome in intensive care unit patients and critically ill patients, wound/skin ulcers/burns healing and treatment, intensification of chemotherapy and/or radiotherapy, pancytopenia, increase of anti-inflammatory citokines, shortening of intervals of high-dose chemotherapy by the prophylactic employment of filgrastim, potentiation of the anti-tumour effects of photodynamic therapy, prevention and treatment of illness caused by different cerebral disfunctions, treatment of thrombotic illness and their complications and post irradiation recovery of erythropoiesis.

It can be also used for treatment of all other illnesses, which are indicative for G-CSF.

The pharmaceutical composition containing the pure and biologically active G-CSF obtained by the process of the invention can thus be administered, in a manner known to those skilled in the art, to patients in a therapeutically amount which is effective to treat the above mentioned diseases.

Preferred embodiments for performing the IMAC in the process according to the present invention will be described in the following.

The process starts with loading of the sample and binding of at least the biologically active form of the G-CSF protein to the IMAC support.

The IMAC support comprises a solid phase material and a metal ion chelate bound to the solid phase material. Conventional solid phase materials can be suitably used, such as Sepharose, Fractogel and other gel support materials. The metal ion chelate bound to the IMAC support is suitably selected from metal ions preferably having two valencies, especially transition metal ions. Hg is less suitable in view of its toxicity and its tendency to be leached out of the IMAC column. Preferred examples of metal ion chelates, being bound to the IMAC support, include: M(II)-iminodiacetate (IDA), M(II)-nitrilotriacetic acid (NTA), M(II)-carboxymethylaspartate etc., where M presents Zn, Cu, Co, Ni etc. Particularly effective are Zn(II) -IDA, Ni(II)-IDA and Ni(II)-NTA.

Before being loaded to the IMAC column, the inclusion bodies preferably are provided as a solution or in the case of batch or expanded bed separation mode, as a suspension in the presence of low detergent or solubiliser concentrations. The G-CSF may thus be kept under native conditions, either when the detergents remain in the solution or suspension or when they are removed by using ion exchangers, dialysis, precipitation, etc. The detergent or solubilising agent is preferably removed before loading the solution or mixture onto the IMAC column.

The inclusion bodies solution or suspension (or mixture) in the presence of strong denaturating agents, such as 8 M urea or 6 M GndHCl, and solutions in the presence of denaturating concentrations of detergents (e.g. 1% sarcosyl, 2% sarcosyl or 1% sodium dodecyl sulfate) can also be used as a starting sample, if the loaded sample had been subjected to a previous renaturation, e.g. by dilution, dialysis, ultrafiltration or removal of denaturation agents/detergents.

In case of the expression in secretory systems such as yeast, fungi or mammalian cell lines, the supernatant or concentrated supernatant or the culture medium, which mixtures may have been processed in advance, with a pH in the range from 6.5 to 9.0 can be loaded to the IMAC support.

The eluate resulting from the first elution from the IMAC column can also be used as loading solution or mixture for IMAC. The pH of the eluate should have been adjusted, e.g. by addition of a NaOH solution or a high pH buffer solution, to the pH range from 6.5 to 9.0. The eluate can be then reloaded to the same IMAC support such as, e.g., Zn(Il)-IDA, Ni(Il)-IDA, Ni(Il)-NTA or to a different IMAC support. The combination with other immobilised metal ions is also possible (e.g. Zn(II), Cu(II), Co(II), Ni(II) etc..) and enables better separation and removal of specific host proteins.

Regardless to the preparation and the origin of the loading solution, the pH of the loading solution should be in the range from 6.5 to 9.0. Preferred pH of loading solution is from 7.0 to 8.5, the mostly preferred pH is from 7.8 to 8.2.

Various buffers, which can maintain pH in the range from 6.5 to 9.0 can be used for loading and binding of G-CSF to the IMAC support. Phosphate, acetate, hydroxymethylaminomethan (Tris)/HCl, Tris/acetate, citrate and other buffers providing a pH of from 6.5 to 9.0 are suitable. Preferably, Tris/HCl is used.

The buffer, especially the Tris/HCl buffer, is preferably used in the concentration range from 5 to 50 mM, most preferably in the range from 10 to 40 mM.

After binding to the support the process is continued by washing of the column and elution of proteins from the column. Elution can be performed by using either a discontinuous step gradient or linear gradient by descending pH or competitive elution at high pH (e.g. with imidazole, histidine, ammonium chloride and similar).

The term 'linear gradient' used herein refers to washing of chromatographic column by a solution which composition is changed in a way that the proportion of one buffer (or one component of the buffer) is increased linearly, whereas the proportion of the other buffer (or another component of the buffer) is decreased linearly.

The term 'discontinuous step gradient' used herein refers to washing of chromatographic column by a solution, which is composed of certain proportion of one buffer (or one component of the buffer) and certain proportion of another buffer (or another component of the buffer) for a determined time period. The proportions of both buffers are rapidly (suddenly) changed and the column is washed by another determined time period. The composition of the solution (change of buffer proportions or component proportions) is not changed linearly.

The term 'competitive elution' used herein refers to elution at the pH of the binding buffer where the competitor molecules, such as imidazole, histidine, ammonium chloride etc., in the elution buffer bind to the metal chelated matrix by themselves and thus displace the protein molecules.

Preferably, discontinuous step gradient, resulting to elution at low pH is used. Namely, high pH could cause the activation of cysteine residues and the formation of dimers. Stability of G-CSF is also high at low pH.

Several elution buffers can be used for discontinuous step or linear washing and elution and are selected from the group consisting of: acetate, Tris/acetate, phosphate, citrate and other suitable buffers. The pH range for the elution can be from 3.0 to 5.0, preferably 3.5 to 4.5. By the discontinuous step gradient, the pH is rapidly changed from the order of the loading pH to the order of elution pH, such as from 7.0 to 4.0, and the isoelectric point is thus jumped over, avoiding the precipitation of the protein. Namely, environment with the pH of the protein isoelectric point can cause its precipitation.

In the eluate, monomeric, biologically active, correctly folded G-CSF is obtained with a purity of greater than 95%.

If desired, the eluate obtained from the IMAC column can be loaded directly to the cationic exchange chromatography column, without any additional intermediate steps being required. Various cationic exchange chromatography supports can be used and may be selected from the group consisting of: SP Sepharose FF, SP Sepharose HP, CM Sepharose FF, TSK gel SP-5PW, TSK gel SP-5PW-HR, Toyopearl SP-650M, Toyopearl SP-650S, Toyopearl SP-650C, Toyopearl CM-650M, Toyopearl CM-650S, Macro-Prep High S support, Macro-Prep S support, Macro-Prep CM support etc. Preferably, SP Sepharose FF or TSK gel SP-5PW are used.

pH range of the loading solution for cationic exchange chromatography is in the range from 3.0 to 5.8, preferably in the range from 4.0 to 5.0.

The salt concentration in the loading solution for cationic exchange chromatography has to be low enough to enable the binding, which also depends on pH of the solution.

Various buffers with the pH range from 3.0 to 5.8 can be used for loading and binding to the support for cationic exchange chromatography and may be selected from the group consisting of: acetate, citrate, Tris/HCl, Tris/acetate, phosphate, succinate, malonate, 2-(N-morfolinoethansulfonate) (MES) and other buffers. Preferably, acetate buffer is used.

Acetate buffer can be used in the concentration range from 10 to 60 mM, preferably in the concentration range from 10 to 30 mM.

In the cationic exchange chromatography, the column loading is followed by washing of the column and the elution of the proteins from the column. The elution occurs due to increased ionic strength after the addition of high concentration of salt in buffer solution. Discontinuous step gradient, linear gradient and a suitable combination of step and linear gradient can be used.

Elution buffers, which can be used for washing and elution, may be selected from the group consisting of: acetate, citrate, Tris/HCl, Tris/acetate, phosphate, succinate, malonate, MES and other suitable buffers with addition of salts such as NaCl or KCI. Ionic strength and salt concentration, by which the elution is achieved, depends on the pH of the buffer solution. The higher is pH of the buffer, the lower ionic strength is needed for the elution of the proteins from the column.

In the eluate, monomeric, biologically active, correctly folded G-CSF is obtained with a purity of greater than 98%.

If desired, the eluate obtained from the IMAC or, preferably, after the consecutive cationic exchange chromatography column can be loaded directly to the gel filtration column, without any additional intermediary steps being required.

Various gel filtration supports can be used and are selected from the group comprising: Sephacryl S-200HR, Sephacryl S-100HR, Superose 12, Superose 6, Superdex 75, TSK gel G-2500PW, TSK gel G-3000 PW, Bio-Gel P-60, Bio-Gel P-100 etc. Preferably, Superdex 75 is used.

A broad pH range of the loading solution for gel filtration can be used and the eluate directly from the IMAC and, preferably, from the subsequent cationic exchange chromatography is therefore suitable as a loading solution. Loading of the solution, binding of the protein to the gel filtration support and elution of the protein can be performed by using the same buffer. Various buffers can be used and may be selected from the group consisting of: citrate, acetate, Tris, phosphate and other suitable buffers, which can maintain pH in the range from 3.5 to 8.0. Preferably, phosphate buffers with pH from 6.0 to 7.0 are used.

Preferably, phosphate buffers (for loading) can be used in the concentration range from 2 to 100 mM, preferably in the concentration range between 3 and 10 mM.

The salt concentrations in the gel filtration buffer can be in the range from 30 to 100 mM, preferably about 50 mM.

In the eluate, monomeric, biologically active, correctly folded G-CSF is obtained with a purity of greater than 99% and biological activity of 1x10⁸ IU/mg.

The following examples are given for the purpose of illustrating various embodiments of the invention and are not meant to limit the present invention in any fashion.

### 1) Description of the drawings

Fig. 1 shows the chromatographic separation of proteins from the solubilized inclusion bodies by using the IMAC support: Zn(II)-IDA Chelating Sepharose fast flow (Pharmacia).
   The chromatogram shows the absorbance change at 280 nm (A280)(-) and the proportion of buffer P3 (-----) in dependence of time (min).
   Peak A -*E*. *coli* proteins and aggregated G-CSF; peak B - monomeric correctly folded biologically active G-CSF and traces of *E. coli* proteins.
Fig. 2 presents the analysis with polyacrylamide gel electrophoresis in the presence of sodium dodecyl sulfate (SDS-PAGE) of the starting sample and the samples represented in chromatographic peaks after the separation by using Zn-IDA Chelating Sepharose fast flow (Pharmacia), according to Fig. 1.
   Legend:
   1. Molecular weight standards (Bio-Rad) and G-CSF (Neupogen) (marked with arrow).
   2. Starting sample before the chromatographic separation.
   3. Proteins in peak A on Fig. 1 (aggregated G-CSF and *E. coli* proteins).
   4. Proteins in peak B on Fig. 1 (monomeric, correctly folded, biologically active G-CSF and traces of *E. coli* proteins).

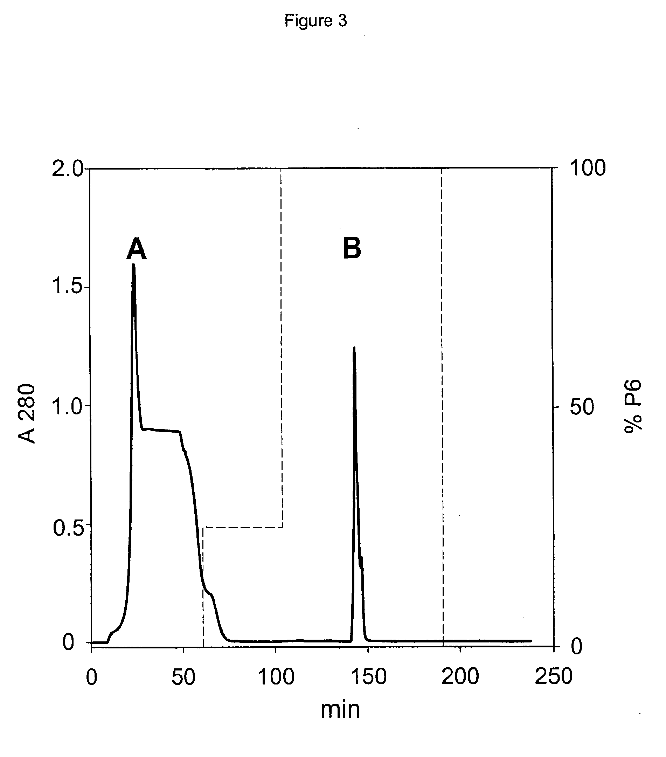

Fig. 3 shows the chromatographic separation by using the column XK50/20, with IMAC support Zn-IDA Chelating Sepharose Fast Flow (Pharmacia).
   The chromatogram shows the absorbance change at 280 nm (A280) (-) and the proportion of buffer P6 (-----) in dependence of time (min).
   Peak A -*E. coli* proteins and aggregated G-CSF; peak B - monomeric correctly folded biologically active G-CSF and traces of *E. coli* proteins.

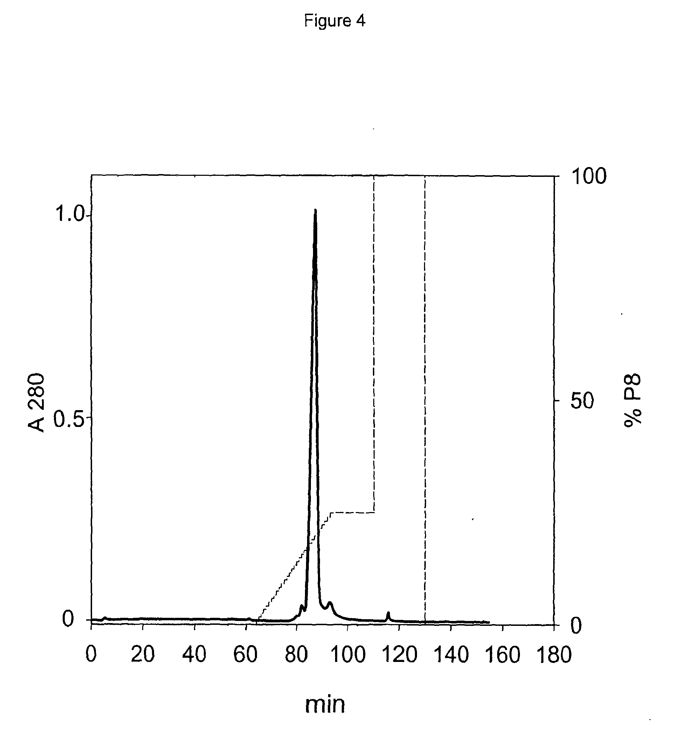

Fig. 4 shows the chromatographic separation by using the column XK16/20, loaded with cationic chromatography support TSK gel SP-5PW (TosoHaas).
   The chromatogram presents the absorbance change at 280 nm (A280) (-) and the proportion of buffer P8 (-----) in dependence of time (min).
   Major peak - monomeric correctly folded biologically active G-CSF; smaller peaks - G-CSF isoforms and traces of *E. coli* proteins.

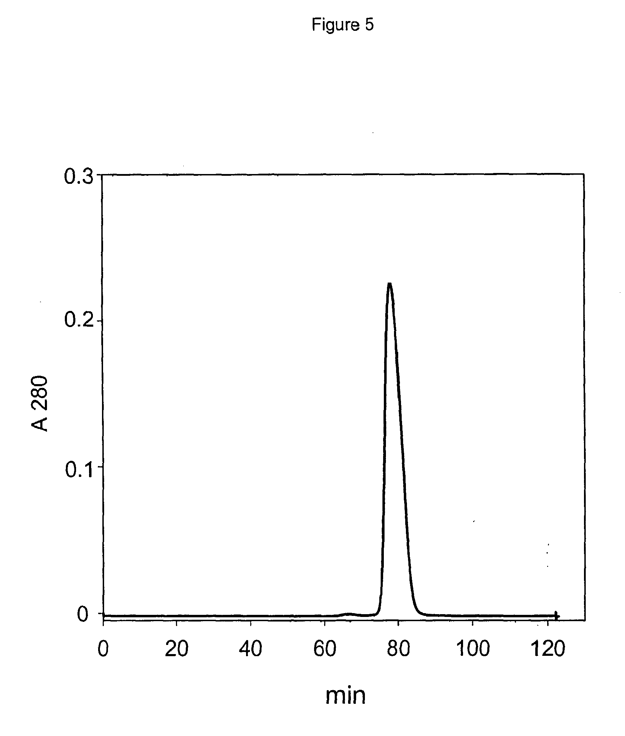

Fig. 5: Chromatographic separation by using the column XK26/70, loaded with gel filtration support Superdex^{™} 75 prep grade (Pharmacia).
   The chromatogram shows the absorbance change at 280 nm (A280) in dependence of (min).
   Major peak - monomeric correctly folded biologically active G-CSF.

### Article II. Examples

### Example 1: The purification and/or isolation of biologically active G-CSF by using IMAC: Zn-IDA (Chelating Sepharose fast flow)

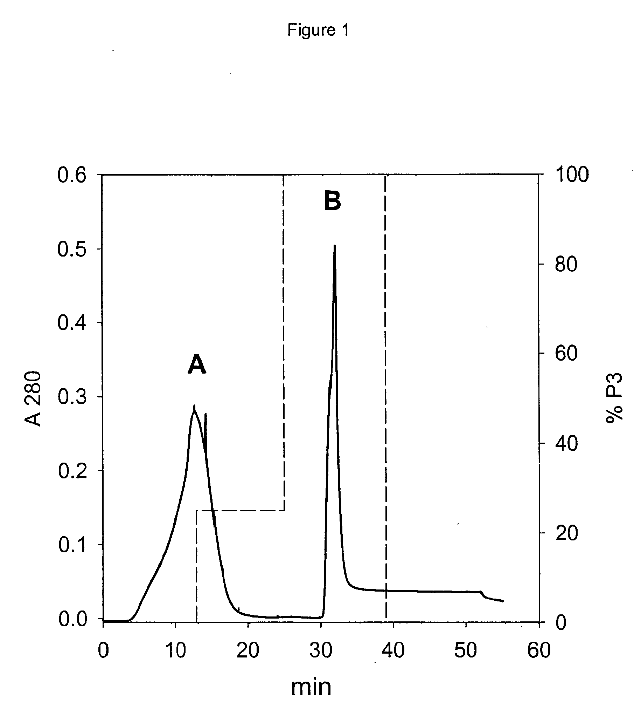

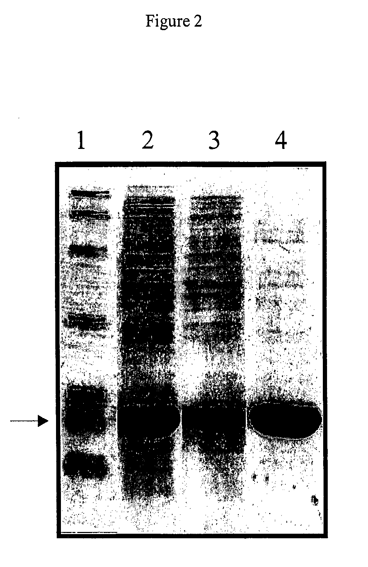

The chromatographic column (h=10 cm, d=10 mm) was loaded with Chelating Sepharose fast flow (Pharmacia) support, on which Zn²⁺ ions were bound, and the column was equilibrated with 5 column volumes of buffer P2 at constant flow of 2 ml/min. Inclusion bodies were solubilized in buffer P1 (0.2 % sarcosyl, 40 mM Tris/HCl, pH 8.0) and sarcosyl was removed by using the ion exchanger. The column was loaded with 10 ml of loading solution (17 mg total proteins), which comprised biologically active G-CSF, at constant flow of 1 ml/min. The separation was performed by using the discontinuous step gradient of buffer P3 at constant flow of 2 ml/min (Fig. 1). In the first chromatographic peak (peak A) the *E. coli* proteins and most of incorrectly folded and aggregated G-CSF were found. At 100% of buffer P3 (0% buffer P2) essentially pure monomeric and biologically active G-CSF was eluted (7 mg) with a purity of greater than 95 % (Fig. 2).

Biological activity of the sample from the peak A was measured as described in Example 5 below and was about 1 x 10⁶ IU/mg proteins, the measured biological activity of the sample from the peak B was 0.8-1.0 x 10⁸ IU/mg proteins, whereas the biological activity of the standard was 1 x 10⁸ IU/mg proteins. One-step IMAC by using the starting inclusion bodies solution leads to the isolation of monomeric form of G-CSF, with a purity of greater than 95 % and the biological activity which is comparable with the standard.

### Example 2: Purification and/or isolation of biologically active G-CSF by using IMAC: Zn-IDA (Fractogel EMD Chelate)

The chromatographic column (h=2 cm, d=10mm) was loaded with Fractogel EMD Chelate (Merck) support on which Zn²⁺ ions were bound. The column was washed with water and equilibrated with five column volumes of buffer P2 at constant flow of 1 ml/min. The inclusion bodies were solubilized in buffer P1 and sarcosyl was removed by using the ion exchanger. The column was loaded with 3,3 ml solubilized inclusion bodies in total amount of 4 mg. The separation was performed by discontinuous step gradient of buffer P3 at constant flow of 1 ml/min (Gradient: 0% buffer P3 (100% buffer P2) 13 min, 25% buffer P3 (75% buffer P2) 12 min, 100% buffer P3 (0% buffer P2) 14 min, 0% buffer P3 (100% buffer P2) 11 min). At 100 % of buffer P3, the monomeric biologically active G-CSF (0,7 mg) was eluted.

### Example 3: Purification and/or isolation of biologically active G-CSF by using IMAC: Ni-NTA (Superflow)

The chromatographic column (h=10 cm, d=10 mm) was loaded with Ni-NTA Superflow (Qiagen) support and was equilibrated with five column volumes of buffer P4 at constant flow of 2 ml/min. The inclusion bodies were solubilized in buffer P1 and the sarcosyl was removed by using the ion exchanger. The column was loaded with 10 ml of solubilized inclusion bodies (10 mg total proteins) at constant flow of 1 ml/min. The separation was performed by using the discontinuous step gradient of buffer P5 at constant flow of 2 ml/min (the same gradient as in separation on Zn-IDA Chelating Sepharose fast flow, presented in Fig. 1). At 100 % of buffer P5 (0% buffer P4) the monomeric biologically active G-CSF (3,6 mg) with a purity of greater than 95% was eluted. In the first chromatographic peak only incorrectly folded and aggregated G-CSF was found in addition to *E. coli* proteins. The monomeric forms of G-CSF from the second peak after several chromatographic separations by using Ni-NTA Superflow were pooled and the final purification (polishing) was performed by using the cationic exchange chromatography and gel filtration.

### Example 4: Process for the purification and/or isolation, including additional chromatographic steps, for the production of biologically active G-CSF

This process for the purification and/or isolation of biologically active G-CSF started from the inclusion bodies solution, which was obtained after the expression of G-CSF in *E*. *coli.* 4,5 g washed inclusion bodies were resuspended in 225 ml of buffer P1 and were left to solubilize at 20°C 18 hours under gentle (light) shaking using the linear shaker. After 15-min of centrifugation the sarcosyl was removed by using the ion exchanger. The sample was diluted with water to reach the double volume and ∼ 430 ml of inclusion bodies solution was obtained; with the protein concentration of ∼ 1,4 mg/ml (after Bradford according to G-CSF as a standard). The protein solution was loaded in two equal volumes to the chromatographic column XK50/20 (Pharmacia), loaded with Chelating Sepharose fast flow (45-165 µm, Pharmacia) support to the height of 10 cm (h=10 cm, d=5 cm, V=200 ml), on which Zn²⁺ ions were bound. The loading of the sample and the elution were performed at constant flow of 7 ml/min. After loading the column was washed with discontinuous step gradient (Fig. 3): 15 min with buffer P2, then 45 min with a mixture of buffers P2 and P6 in the volume ratio of 75:25 and 86 min with buffer P6. Monomeric form of biologically active G-CSF was eluted at 100% buffer P6. All the fractions (two separations), which comprised the monomeric biologically active G-CSF were pooled and 271 ml of solution with protein concentration of ∼ 0,7 mg/ml was obtained. EDTA was added to this solution to the final concentration of 2 mM. The solution was diluted three times with 20 mM CH₃COOH, pH 4,0 and was used as a loading solution for cationic exchange chromatography.

The IMAC eluate was loaded in two aliquots to the chromatographic column XK16/20 (Pharmacia), loaded with chromatographic support SP-5PW (30 µm; TosoHaas) to the height of 16 cm (h=16 cm, d=1,6 cm, V=32 ml). The loading of the sample and the elution from the column were performed at constant flow of 5 ml/min. After the loading of the sample the column was washed 11 min with buffer P7, followed by the elution with linear gradient with buffer P8 in 30 min from 0% to 25 % buffer P8 (from 100% to 75% buffer P7). The column was washed again for 16 min with the mixture of buffers P7 and P8 in a volume ratio of 75:25 and then 22 min with buffer P8 (Fig. 4). The fractions of the main chromatographic peak, which were eluted in a linear part of the linear gradient at ∼ 18% of buffer P8 and belonged to the correctly folded G-CSF (with a purity of greater than 98%), were pooled and used directly as a loading solutions for the gel filtration column.

The eluate from the cationic exchange chromatography (V= 46 ml, protein concentration ∼ 2,4 mg/ml), was loaded in 5 aliquots to the chromatographic column XK26/70 (Pharmacia), loaded with the gel filtration support Superdex 75 (prep grade, 34 µm) (Pharmacia) to height 57 cm (h=57 cm, d=2.6 cm, V=300 ml). The separation was performed in buffer P9 at constant flow of 2,5 ml/min. Peak, which represents the protein dimer, is clearly separated from the main peak which represents the monomeric protein (Fig. 5). The main chromatographic peak fractions were pooled, the buffer was changed and 100 mg of pure monomeric form of G-CSF with a purity of greater than 99% and biological activity of 1 x 10⁸ IU/mg, which corresponds to the biological activity of the standard, was obtained.

### (a) Example 5: In vitro G-CSF biological activity assay

Biological activity of G-CSF was determined by the method based on stimulation of cellular proliferation (NFS-60 cells) using the known method (Hammerling, U. et al. in J Pharm Biomed Anal 13, 9-20 (1995)) and the use of international standard Human recombinant G-CSF (88/502, yeast cell derived; NIBSC Potters Bar, Hertfordshire, UK; see Mire-Sluis,A.R. et al. v J Immunol Methods 179, 117-126 (1995)

**The buffer compositions:**
P1: 0,2% sarcosyl, 40 mM Tris/HCl, pH 8,0
P2: 20 mM Tris/HCl, 150 mM NaCl pH 8,0
P3: 20 mM acetic acid, 150 mM NaCl, pH adjusted to 4,5 with addition of 1 M NaOH
P4: 10 mM Tris/HCl, 200 mM NaCl, pH 8,0
P5: 20 mM acetic acid, 200 mM NaCl, pH adjusted to 4,0 with addition 1 M NaOH
P6: 20 mM CH₃COOH, 150 mM NaCl, pH 4,0
P7: 20 mM CH₃COOH, pH 5,5
P8: 20 mM CH₃COOH, 500 mM NaCl, pH 5,5
P9: 5 mM Na phosphate, 50 mM NaCl, pH 7,0

### Further exemplary embodiments

[1.]A process for the purification and/or isolation of biologically active G-CSF, which comprises:
   providing a mixture which comprises a biologically active form of G-CSF in the presence of an impurity, and
   subjecting said mixture to IMAC.
[2.]The process for the isolation of biologically active G-CSF according to claim 1, which comprises the following steps:
   - loading said mixture, which comprises the biologically active G-CSF in the presence of an impurity, to an IMAC support,
   - selective binding of the biologically active form of G-CSF to the IMAC support and
   - eluting the biologically active form of G-CSF from the IMAC support to provide the biologically active G-CSF.
[3.] The process according to embodiment 1 or 2, wherein said mixture comprises an impurity which is at least one substance among the group consisting of
   biologically inactive monomeric forms and incorrectly folded molecules of G-CSF, oligomeric and polymeric forms of G-CSF, denaturated forms of G-CSF, host cell proteins and other host cell impurities (components);
   and the IMAC is carried out in such a way that the impurity is substantially not bound to the IMAC support and eluted from the IMAC column, before eluting the biologically active form of G-CSF.
[4.]The process according to any one of embodiments 1 to 3, wherein the biologically active G-CSF is at least one selected from the group consisting of non-glycosylated G-CSF, glycosylated G-CSF, methionyl G-CSF, G-CSF analogues, enzymatically or chemically modified forms of G-CSF and fusion proteins which comprise G-CSF.
[5.] The process according to any one of the preceding embodiments, wherein said G-CSF-containing mixture is selected from the group consisting of:
   a mixture, medium or solution, obtained after denaturation followed by renaturation;
   a solution or suspension of inclusion bodies under native conditions;
   a mixture or solution obtained from the supernatant after the expression in secretory systems or from a culture medium of an expression system;
   and an eluate which was obtained by a previous elution of G-CSF from an IMAC column or any other chromatographic column.
[6.]The process according to embodiment 1 or 5, wherein said mixture comprises an inclusion body solution or suspension under native conditions.
[7.]The process according to any one of the preceding embodiments, wherein an IMAC with chelated metal ion bound to the IMAC support is carried out, wherein the metal ion is not Hg and/or wherein the chelated metal ion bound to the IMAC support is selected from the group consisting of M(II)-iminodiacetate, M(II)-nitrilotriacetic acid and M(II)-carboxymethylaspartate, wherein M is selected from the group consisting of Zn, Cu, Co and Ni.
[8.]The process according to embodiment 7, wherein the chelated metal ion bound to the IMAC support is selected from the group consisting of Zn(II)-iminodiacetate, Ni(II)-iminodiacetate and Ni(II)-nitrilotriacetic acid.
[9.]The process according to any one of the preceding embodiments, wherein the biologically active G-CSF obtained after performing IMAC has a purity of at least 95%.
[10.] The process according to any one of the preceding embodiments, further comprising the step(s) of: cationic exchange chromatography and/or gel filtration chromatography.
[11.] The process according to embodiment 10, wherein the biologically active G-CSF obtained after performing the chromatographic steps has a purity of at least 99%.
[12.] A process for the purification and/or isolation of biologically active G-CSF, wherein an impure mixture containing G-CSF under native conditions is subjected to chromatographic step(s) which consist only of IMAC, and optionally at least one of the purification methods selected from ion exchange and gel filtration techniques.
[13.] The process according to embodiment 12, wherein the mixture containing G-CSF is essentially free of detergent or solubilising agent or contains detergent or solubilising agent at a concentration where the G-CSF is present under native conditions.
[14.] The process according to embodiment 12 or 13, wherein the biologically active G-CSF obtained after performing the chromatographic steps has a purity of at least 95%; preferably at least 99%.
[15.] The process according to any one of the preceding embodiments, wherein the whole process is performed under native conditions.
[16.] A biologically active G-CSF with a purity of greater than 99%.
[17.] The biologically active G-CSF according to embodiment 16, as obtained by a process defined by any one of the embodiments 1 to 15.
[18.] A pharmaceutical composition comprising a therapeutically effective amount of biologically active G-CSF with a purity of greater than 99% and pharmaceutically acceptable auxiliary substances.
[19.] The pharmaceutical composition according to embodiment 18, wherein the biologically active G-CSF is obtained by a process as defined in any one of the embodiments 1 to 15.
[20.] The use of the biologically active G-CSF as obtained by a process, defined by any one of embodiments 1 to 15, for the production of medicaments for indications selected from the group consisting of: neutropenia and neutropenia-related clinical sequelae, reduction of hospitalisation for febrile neutropenia after chemotherapy, mobilisation of hematopoietic progenitor cells as alternative to donor leukocyte infusion, chronic neutropenia, neutropenic and non-neutropenic infections, transplant recipients, chronic inflammatory conditions, sepsis and septic shock, reduction of risk, morbidity, mortality, number of days of hospitalisation in neutropenic and non-neutropenic infections, prevention of infection and infection-related complications in neutropenic and non-neutropenic patients, prevention of nosocomial infection and to reduce the mortality rate and the frequency rate of nosocomial infections, enteral administration in neonates, enhancing the immune system in neonates, improving the clinical outcome in intensive care unit patients and critically ill patients, wound/skin ulcers/burns healing and treatment, intensification of chemotherapy and/or radiotherapy, pancytopenia, increase of anti-inflammatory cytokines, shortening of intervals of high-dose chemotherapy by the prophylactic employment of filgrastim, potentiation of the anti-tumour effects of photodynamic therapy, prevention and treatment of illness caused by different cerebral disfunctions, treatment of thrombotic illness and their complications and post irradiation recovery of erythropoiesis.

## Claims

1. A process for the purification and/or isolation of biologically active G-CSF, which comprises:
providing a mixture which comprises a biologically active form of G-CSF in the presence of an impurity, and
subjecting said mixtures to IMAC.

2. The process for the isolation of biologically active G-CSF according to claim 1, which process comprises the following steps:
• loading said mixture, which comprises the biologically active G-CSF in the presence of an impurity, to an IMAC support,
• selective binding of the biologically active form of G-CSF to the IMAC support and
• eluting the biologically active form of G-CSF from the IMAC support to provide the biologically active G-CSF.

3. The process according to claim 1 or 2, wherein said mixture comprises an impurity which is at least one substance among the group consisting of biologically inactive monomeric forms and incorrectly folded molecules of G-CSF, oligomeric and polymeric forms of G-CSF, denaturated forms of G-CSF, host cell proteins and other host cell impurities (components);
and the IMAC is carried out in such a way that the impurity is substantially not bound to the IMAC support and eluted from the IMAC column, before eluting the biologically active from of G-CSF.

4. The process according to any one of claims 1 to 3, wherein the biologically active G-CSF is at least one selected from the group consisting of non-glycosylated G-CSF, glycosylated G-CSF, methionyl G-CSF, G-CSF analogues, enzymatically or chemically modified forms of G-CSF and fusion proteins which comprise G-CSF.

5. The process according to claim 4, wherein the biologically active G-CSF is non-glycosylated G-CSF.

6. The process according to any one of the preceding claims, wherein said G-CSF-containing mixture is selected from the group consisting of:
a mixture, medium or solution, obtained after denaturation followed by renaturation;
a solution or suspension of inclusion bodies under native conditions;
a mixture or solution obtained from the supernatant after the expression in secretory systems or from a culture medium of an expression system;
and an eluate which was obtained by previous elution of G-CSF from an IMAC column or any other chromatographic column.

7. The process according to claim 1 or 6, wherein said mixture comprises an inclusion body solution or suspension under native conditions.

8. The process according to any one of the preceding claims, wherein an IMAC with chelated metal ion bound to the IMAC support is carried out, wherein the metal ion is not Hg and/or wherein the chelated metal ion bound to the IMAC support is selected from the group consisting of M(II)-iminodiacetate, M(II) nitrilotriacetic acid and M(II)-carboxymethylaspartate, wherein M is selected from the group consisting of Zn, Cu, Co and Ni.

9. The process according to claim 7, wherein the chelated metal ion bound to the IMAC support is selected from the group consisting of Zn(II)-iminodiacetate, Ni(II)-iminodiacetate and Ni(II)-nitrilotriacetic acid.

10. The process according to any one of the preceding claims, further comprising the step(s) of cationic exchange chromatography and/or gel filtration chromatography.

11. A process for the purification and/or isolation of biologically active G-CSF, wherein an impure mixture containing G-CSF under native conditions is subjected to chromatographic step(s) which consist only of IMAC, and optionally at least one of the purification methods selected from ion exchange and gel filtration techniques.

12. The process according to claim 11, wherein the mixture containing G-CSF is essentially free of detergent or solubilising agent or contains detergent or solubilising agent at a concentration where the G-CSF is present under native conditions.

13. The process according to any one of the preceding claims, wherein the whole process is performed under native conditions.

14. The process according to any one of the preceding claims, further comprising the step of using the G-CSF obtained for preparation of a medicament.
